# DEMANDE DE BREVET EUROPEEN

(11) **EP 3 159 010 A1**
(43) Date de publication de la demande: **26.04.2017**
(21) Numéro de dépôt: 16002253.9
(22) Date de dépôt: 20.10.2016
(51) Int. Cl.: A61K 45/06, A61K 36/48, A61K 36/71, A23L 35/00, A23L 33/105

(54) **NOUVELLE COMPOSITION ALIMENTAIRE HYPOCHOLESTÉROLÉMIANTE**

(30) Priorité: 20.10.2015 FR 1502211
(71) Demandeur: OTX SOLUTIONS, 8362 Grass (LU)
(72) Inventeur: Zintz, Benoit, 8041 Strassen (LU)
(74) Mandataire: Barbot, Willy

(57) **Abrégé**

La présente invention concerne une composition comestible comprenant a) au moins un extrait au CO₂ supercritique de *Nigella sativa ;* et b) au moins un extrait au CO₂ supercritique de *Trigonella foenum-graecum,* laquelle est avantageusement destinée au traitement de l'hypercholestérolémie chez un sujet.

## Description

### Domaine de L'invention

La présente invention est relative à des compositions permettant d'abaisser le niveau total de cholestérol.

### Art antérieur de l'invention

Le cholestérol est un lipide de la famille des stérols dont il est certainement le représentant le plus important chez l'animal, où on le trouve dans tous les tissus et particulièrement dans le cerveau.

Ce composant est un composant fondamental des membranes cellulaires animales, dont il contrôle la stabilité en s'intercalant entre les phospholipides. En effet, le cholestérol rigidifie la membrane en empêchant sa gélification car il évite la cristallisation des acides gras et diminue également la perméabilité membranaire aux molécules hydrosolubles. En s'intercalant dans les membranes, le cholestérol permet également la formation de radeaux lipidiques, essentiels à l'ancrage de protéines fonctionnelles.

Le métabolisme du cholestérol est également précurseur de nombreuses molécules comme par exemple les hormones stéroïdes (cortisol, cortisone et aldostérone), les hormones stéroïdes sexuelles (progestérone, oestrogènes et testostérone), la vitamine D3, l'hème A, les protéines prénylées ou farnésylées, l'ubiquinone ou coenzyme Q10, le dolichol, le facteur nucléaire NF kappa B, la protéine Tau et les sels biliaires.

Le cholestérol est véhiculé dans le sang par des systèmes de transport aux rôles très différents, à savoir les lipoprotéines LDL (lipoprotéines de petite densité) et les lipoprotéines HDL (lipoprotéines de haute densité).

Le HDL-cholestérol est considéré comme protecteur vis-à-vis des maladies cardiovasculaires et est fréquemment désigné comme « bon cholestérol ».

Les LDL transportant le cholestérol pénètrent dans la paroi artérielle puis sont captées par les monocytes et macrophages résidant dans cette paroi artérielle ainsi que par les cellules musculaires lisses. Il s'ensuit une charge excessive en lipides des macrophages et cellules musculaires lisses qui se transforment alors en cellules spumeuses à l'origine des processus d'athérosclérose et correspondant au premier stade de la formation de la plaque d'athérome. Il est alors question de « mauvais cholestérol ».

L'athérome est défini par l'Organisation Mondiale de la Santé comme une "association de remaniement de l'intima des artères de gros et moyen calibre consistant en une accumulation focale de lipides, glucides complexes, de sang et de dépôts calcaires, avec remaniements de la média. L'athérome est à l'origine de la majorité des maladies cardiovasculaires et est la principale cause de morbidité et de mortalité des pays industrialisés. L'athérome débute par la formation d'une « strie lipidique », simple dépôt de graisse, linéaire et sans conséquence pour le flux, située entre endothélium et média de l'artère. Avec le temps, cette strie peut grossir, se charger en lipides, en fibrogène, en plaquettes et autres cellules sanguines et en calcium pour constituer la « plaque d'athérome ». Celle-ci devient plus ou moins importante et peut diminuer suffisamment le calibre de l'artère pour diminuer son débit.

Il existe deux types de complications : la première, d'évolution longue, est due à sa croissance lente, gênant de plus en plus le passage du sang jusqu'à l'empêcher totalement par obstruction. La seconde, rapide et responsable des complications aiguës, consiste en la lésion ou la rupture de l'endothélium : la brèche formée est alors obstruée par une agrégation des plaquettes sanguines et la formation d'un caillot sanguin qui peut rapidement totalement obstruer le vaisseau. Ce caillot peut également se détacher et obstruer plus en aval. La plaque peut également se détacher partiellement et obstruer également l'artère, ou plus rarement, libérer son contenu et faire une embolie de cholestérol. Enfin, la dilatation de la paroi artérielle induite par l'augmentation de volume de la plaque peut aboutir à la formation d'un anévrisme, avec risque de rupture.

La classe pharmaceutique la plus employée pour prévenir l'athérome est celle des statines, qui visent à réduire le taux de cholestérol-LDL et de lipides circulants, en complément d'un régime adapté. Cependant l'intérêt et l'utilité des statines sont depuis quelques temps vivement critiqués par certains chercheurs et cliniciens qui affirment que les statines, après analyse d'essais statistiques controversés car contradictoires, n'apportent pas aux patients le bénéfice thérapeutique escompté. A cette mise en cause s'ajoutent les effets secondaires néfastes, et même parfois dramatiques, qui ont été identifiés et ont conduit dans certains cas à un retrait du marché.

Les autres traitements médicamenteux proposés aujourd'hui sont :
- les antiagrégants plaquettaires comme l'aspirine ou le CLOPIDOGREL, censés diminuer la constitution de caillots à partir de la plaque d'athérome ;
- les médicaments antihypertenseurs tels que les inhibiteurs de l'enzyme de conversion de l'angiotensine.

Les traitements pharmaceutiques qui existent à l'heure actuelle visent donc uniquement à diminuer les risques liés à l'athérome. Aucun des médicaments existant sur le marché ne s'attaque directement à la plaque d'athérome.

Lorsque le pronostic vital est en jeu, le traitement est chirurgical ou endovasculaire. Il vise à restaurer la lumière artérielle, à irriguer le territoire privé d'oxygène ou à supprimer l'anévrisme artériel. Parmi ces techniques, on peut citer l'angioplastie, la désobstruction par endartériectomie, le pontage.

Finalement, le principal moyen de lutte contre un niveau élevé de cholestérol et l'apparition de l'athérome et de ses complications reste encore aujourd'hui d'ordre comportemental : arrêt du tabac, développement de l'activité physique, contrôle de la tension artérielle, correction d'une dyslipidémie, équilibre du diabète, régime.

Ainsi, force est de constater qu'il existe clairement et depuis longtemps, un besoin non satisfait d'abaisser le niveau de cholestérol.

Finalement, il existe toujours sur le marché un besoin de composition capable d'abaisser le niveau de cholestérol et, par ricochet, de lutter contre l'athérosclérose, en particulier contre la formation de la plaque d'athérome.

### Descriptif de l'invention

Les inventeurs ont pu mettre en évidence que l'administration orale, chez des sujets, de gélules présentant une composition, comprenant tout à la fois un extrait au CO₂ supercritique de *Nigella sativa* et un extrait au CO₂ supercritique de *Trigonella foenum-graecum,* permettait d'abaisser sensiblement le niveau de cholestérol total et, par là même de lutter contre l'athérosclérose.

On connaît de la demande de brevet NL 1034499 une composition comprenant un extrait de *Nigella sativa* et de *Trigonella foenum-graecum.* Maintenant, il n'est jamais fait mention d'extraits au CO₂ supercritique, dont la composition est naturellement particulière du fait de ce procédé spécifique, pas plus que de l'effet étonnant de celle-ci sur l'abaissement du cholestérol total et donc sur les risques d'athérosclérose.

En conséquence, un premier objet de l'invention porte sur une composition comestible comprenant :
- au moins un extrait au CO₂ supercritique de *Nigella sativa*; et
- au moins un extrait au CO₂ supercritique de *Trigonella foenum-graecum*.

Les inventeurs ont pu mettre en évidence qu'une telle composition comestible, du fait de la nature lipophile des extraits utilisés permettait de bénéficier d'une très bonne biodisponibilité des composés actifs s'y trouvant et également d'obtenir une très bonne activité hypocholestérolémiante.

De surcroît, les utilisateurs ont apprécié les qualités organoleptiques de la composition selon l'invention, alors même qu'elle contient un extrait de fenugrec, ce qui a grandement facilité sa prise quotidienne, laquelle prise quotidienne est bien évidemment nécessaire pour produire ses effets avec le temps.

*Nigella sativa*, communément dénommée nigelle cultivée ou cumin noir, est une plante annuelle de la famille des Renonculacées originaire du sud-ouest de l'Asie. Ses graines sont utilisées comme remède traditionnel ou comme épice dans de nombreux pays du monde et particulièrement dans le Moyen-Orient. Si ces graines sont potentiellement toxiques en raison de la présence de terpénoïdes et d'alcaloïdes, elles peuvent toutefois être consommées sans danger en petites quantités.

De préférence, l'extrait utilisé est un extrait de graines de *Nigella sativa*.

A titre d'exemple, on peut citer l'extrait de CO₂ supercritique de *Nigella sativa* disponible chez FLAVEX (122.002).

La composition selon l'invention comprend une teneur comprise entre 100 à 1 000 mg d'extrait au CO₂ supercritique de *Nigella sativa*, de préférence comprise entre 200 et 800 mg, et de préférence encore comprise entre 300 et 500 mg d'extrait au CO₂ supercritique de *Nigella sativa*.

*Trigonella foenum-graecum,* communément dénommée Fenugrec, Trigonelle ou Sénégrain, est une plante herbacée de la famille des *Fabacées*, qui est utilisé principalement comme plante médicinale et condimentaire. Cette plante bénéficie de nombreuses propriétés médicinales avérées parmi lesquelles une activité hypocholestérolémiante avec la réduction du taux de cholestérol et des triglycérides. Maintenant, son odeur caractéristique est problématique pour son absorption.

De préférence là encore, l'extrait utilisé est un extrait de graines de *Trigonella foenum-graecum*.

A titre d'exemple, on peut citer l'extrait de CO₂ supercritique de *Trigonella foenum-graecum* disponible chez FLAVEX (088.003).

La composition selon l'invention comprend une teneur comprise entre 10 et 500 mg d'extrait au CO₂ supercritique de *Trigonella foenum-graecum,* de préférence entre 10 et 100 mg ou entre 50 à 500 mg d'extrait au CO₂ supercritique de *Trigonella foenum-graecum,* de préférence comprise entre 20 et 80 mg ou entre 100 et 400 mg, et de préférence encore comprise entre 30 et 70 mg ou entre 150 et 350 mg d' extrait au CO₂ supercritique de *Trigonella foenum-graecum*.

Chacun de ces deux extraits au CO₂ supercritique est lipophile, à l'état solide ou semi-solide à température ambiante, et également incolore ou faiblement coloré. Cette absence de coloration est liée aux conditions expérimentales du procédé d'extraction qui évitent l'entrainement d'une trop grande quantité de pigments présents dans les tissus végétaux. La nature des extraits utilisés, leur permet tout à la fois une concentration des principes actifs le composant au regard de la plante entière et une absence de solvants organiques, lesquels présentent souvent des effets indésirables (ex. toxicité et/ou irritation).

On définit l'état supercritique pour un fluide, comme étant l'état dans lequel se trouve ce fluide quand on le soumet à des conditions de température et de pression telles que la température appliquée est supérieure à une température critique (Tc) et la pression appliquée est supérieure à une pression critique (Pc), ces valeurs critiques étant spécifiques à chaque fluide.

Pour le dioxyde de carbone, qui constitue en l'espèce le solvant pour l'extraction, la température critique Tc est égale à 31 °C et la pression critique Pc est égale à 7,38 x 10⁶ Pa.

Pour l'obtention d'un extrait au CO₂ supercritique, on utilisera de préférence :
- une pression comprise entre 8 x 10⁶ Pa et 30 x 10⁶ Pa ; et
- une température comprise entre 35 et 80°C.

En fin d'extraction, l'extrait utilisé a subi une phase dite de détente, par abaissement de la pression et éventuellement de la température, qui provoque le passage du dioxyde de carbone de l'état supercritique à l'état gazeux, et qui permet d'obtenir un extrait dont a été éliminé complètement le dioxyde de carbone.

Eventuellement, l'extrait utilisé a également été déshydraté, de sorte d'éliminer toute trace d'eau résiduelle. Typiquement cette déshydratation a été opérée par une opération de lyophilisation de l'extrait obtenu.

Avantageusement, la composition selon l'invention comprend en outre au moins un extrait de romarin.

Le Romarin ou Romarin officinal *(Rosmarinus officinalis),* est un arbrisseau de la famille des Lamiacées (ou labiées), poussant à l'état sauvage sur le pourtour méditerranéen.

L'effet antioxydant de cet extrait permet, en améliorant la stabilité de la composition de l'invention, de potentialiser encore l'activité de celle-ci. Un tel extrait est notamment disponible là encore chez FLAVEX (027.002, 027.003, 027.004, 027.010, 027.020, 027.025, 027.032, 148.001 ou 148.002).

Le pourcentage en poids d'extrait de romarin pour la composition selon l'invention est avantageusement compris entre 0,005% et 0,5%, de préférence entre 0,01% et 0,1%.

Avantageusement encore, la composition selon l'invention se présente sous la forme d'une gélule, de préférence d'une gélule végétale.

Une telle gélule notamment végétale peut être réalisée simplement, notamment avec une enveloppe à base de cellulose (Hydroxypropylmethylcellulose, ou « HPMC », ou encore « Hypromellose ») ; laquelle cellulose peut être additionnée de colorants naturels, de sorte d'obtenir l'enveloppe présentant les propriétés souhaitées.

De préférence, ladite gélule présente une couleur jaune ou verte.

Idéalement, la gélule selon l'invention a une masse comprise entre 500 et 1 000 mg pour permettre une bonne ingestion, de préférence entre 800 et 900 mg.

Ladite enveloppe peut en outre également comprendre des agents opacifiants tels que l'oxyde de titane.

D'autres agents pharmaceutiquement et/ou alimentairement acceptables peuvent être rajoutés, tels que des agents antioxydants, des agents de charge, des fluidisants, des extraits naturels, des minéraux, des oligoéléments, des acides aminés, des acides gras, des huiles naturelles, des arômes, des colorants, des acidifiants, des épaississants, des conservateurs et des édulcorants.

A titre d'exemples de tels agents antioxydants, on pourra citer, les polyphénols, notamment sous la forme d'extraits végétaux (extraits de thé vert, de raisin, ginseng), la vitamine C, notamment sous la forme d'extraits végétaux (extrait d'acérola, de grenade, d'agrumes), ou encore la vitamine E, notamment sous forme d'extraits végétaux ; ou leur dérivés.

A titre d'exemples d'agents de charge, on pourra citer la cellulose microcristalline, la maltodextrine de pomme de terre ou encore le lactate de magnésium.

A titre d'exemples de fluidisants, on pourra citer le silicate de magnésium, le stéarate de magnésium ou encore la silice colloïdale.

A titre d'exemples d'extraits naturels, on pourra citer les extraits de thé vert, de cannelle, de guarana, de maté, de fenouil, de reine des prés, de maïs, de sauge, de mélisse ou encore de café.

A titre d'exemples de minéraux ou d'oligoéléments, on pourra citer le magnésium, l'iode, le fer, le cuivre, le zinc, le sélénium, le chrome, le molybdène,- le manganèse, le silicium, le vanadium, le nickel ou encore l'étain.

A titre d'exemples d'acides aminés, on pourra citer l'alanine, la cystéine, l'acide aspartique, l'acide glutamique, la phénylalanine, la glycine, l'histidine, l'isoleucine, la lysine, la leucine, la méthionine, l'asparagine, la proline, la glutamine, l'arginine, la sérine, la thréonine, la valine, le tryptophane ou la tyrosine.

A titre d'exemples d'acide gras, on pourra citer les acides gras insaturés tels que les oméga-3 ou les oméga-6.

A titre d'exemple d'épaississant, on pourra citer l'amidon de pomme de terre, l'hydroxypropylméthylcellulose, la pectine de citrus, la gomme de guar, de caroube, l'agar-agar, le konjac, les huiles hydrogénées ou encore la cire d'abeille.

A titre d'exemple d'acidifiants, on pourra citer l'acide citrique.

A titre d'exemples d'édulcorants, on pourra citer, entre autres, le xylitol, l'aspartame, le sirop de glucose, le sirop de fructo-oligosaccharide, le maltitol en poudre ou en sirop, l'acésulfame de potassium, le fructooligosaccharide et le cyclamate de sodium.

Un second objet de l'invention porte sur l'utilisation d'une composition telle que décrite précédemment pour le traitement de l'hypercholestérolémie chez un sujet.

Par sujet, on entend un humain: un homme ou une femme.

Par hypercholestérolémie (littéralement : cholestérolémie élevée) on entend un taux élevé de cholestérol sanguin correspondant à un trouble métabolique.

Maintenant, le traitement de l'hypercholestérolémie permet la prévention de l'athérome.

Les exemples ci-après sont fournis pour illustrer l'invention et ne doivent en aucun cas être considérés comme une limite à la portée de l'invention.

### 1) Préparation de gélules

On prépare des gélules d'hydroxypropylmethylcellullose (HPMC) comprenant la composition décrite dans le tableau ci-après.

| Ingrédients | Proportion |
|---|---|
| extrait au CO₂ supercritique de *Nigella sativa* | 400 à 600 mg |
| extrait au CO₂ supercritique de *Trigonella foenum-graecum* | 30 à 70 mg |
| Extrait de romarin | 300 à 500 ppm |

### 2) Evaluation de l'efficacité sur la phase l'hypercholestérémie

L'efficacité de la composition selon l'invention a été testée sur un panel de plusieurs dizaines de personnes.

Les personnes du panel ont ingéré quotidiennement une à deux gélules de la composition selon l'invention.

On a observé un abaissement du cholestérol total et des triglycérides.

On a également observé un abaissement de l'apolipoprotéine B, de la ferritine et de la CRP (protéine C réactive) et, parallèlement une augmentation de l'apolipoprotéine A1.

En outre, les personnes du panel ont apprécié les propriétés organoleptiques de la composition, ce qui a permis une très bonne observance de leur part.

Finalement, ces résultats établissent les propriétés intéressantes de la composition selon l'invention.

## Revendications

1. Une composition comestible comprenant :
a) au moins un extrait au CO₂ supercritique de *Nigella sativa ;* et
b) au moins un extrait au CO₂ supercritique de *Trigonella foenum-graecum*.

2. La composition selon la revendication 1, **caractérisée en ce que** l'extrait au CO₂ supercritique de *Nigella sativa* utilisé est un extrait de graines de *Nigella sativa*.

3. La composition selon l'une quelconque des revendications précédentes, **caractérisée en ce que** la teneur de l'extrait au CO₂ supercritique de *Nigella sativa* est comprise entre 100 à 1 000 mg.

4. La composition selon l'une quelconque des revendications précédentes, **caractérisée en ce que** l'extrait au CO₂ supercritique de *Trigonella foenum-graecum* utilisé est un extrait de graines de *Trigonella foenum-graecum*.

5. La composition selon l'une quelconque des revendications précédentes, **caractérisée en ce que** la teneur de l'extrait au CO₂ supercritique de *Trigonella foenum-graecum* est comprise entre 10 à 500 mg.

6. La composition selon l'une quelconque des revendications précédentes, **caractérisée en ce qu'**elle comprend en outre au moins un extrait de romarin.

7. La composition selon la revendication 6, **caractérisée en ce que** le pourcentage en poids d'extrait de romarin (par rapport au poids total de la composition) est compris entre 0,005% et 0,5%, de préférence entre 0,01 % et 0,1 %.

8. La composition selon l'une quelconque des revendications précédentes, **caractérisée en ce qu'**elle se présente sous la forme d'une gélule, de préférence d'une gélule végétale.

9. Une composition selon l'une quelconque des revendications 1 à 8 destinée au traitement de l'hypercholestérolémie chez un sujet.

10. Une composition selon la revendication précédente, **caractérisée en ce qu'**elle est destinée à la prévention de l'athérome.
